# EUROPÄISCHE PATENTSCHRIFT

(11) **EP 2 254 619 B1**
(45) Veröffentlichungstag und Bekanntmachung des Hinweises auf die Patenterteilung: **06.06.2012**
(21) Anmeldenummer: 09712372.3
(22) Anmeldetag: 20.02.2009
(51) Int. Cl.: A61M 1/34, A61M 1/36

(54) **BIOÄQUIVALENZDIALYSE**
BIOEQUIVALENCE DIALYSIS
DIALYSE DE BIOÉQUIVALENCE

(30) Priorität: 22.02.2008 DE 102008010691
(43) Veröffentlichungstag der Anmeldung: 01.12.2010
(73) Patentinhaber: Universität Rostock, 18055 Rostock (DE)
(72) Erfinder: MITZNER, Steffen, 18055 Rostock (DE)
(74) Vertreter: UEXKÜLL & STOLBERG
(86) Internationale Anmeldenummer: PCT/EP2009/001233
(87) Internationale Veröffentlichungsnummer: WO 2009/103553

(56) Entgegenhaltungen:
- WO-A-01/51068
- WO-A-2007/046757

## Beschreibung

Die vorliegende Erfindung betrifft ein Verfahren zur Behandlung von Blutkompositionsstörungen und Blutfunktionsstörungen. Dieses umfasst Schritte, bei denen man Blut oder Blutplasma eines Patienten extrakorporal mit einer zweiten Flüssigkeit (Bioäquivalent) in Kontakt bringt, wobei die zweite Flüssigkeit Blut oder modifiziertes Blut ist, welches mindestens Granulozyten, Thrombozyten und Erythrozyten umfasst. Die Erfindung betrifft ferner Vorrichtungen zur Durchführung dieses Verfahrens und die Verwendung einer solchen Vorrichtung oder von Blut oder modifiziertem Blut, welches Granulozyten, Thrombozyten und Erythrozyten umfasst, zur Behandlung von Blutkompositionsstörungen und Blutfunktionsstörungen.

Blut ist ein komplexes Organ, welches viele verschiedene, miteinander und mit anderen körpereigenen Systemen interagierende zelluläre Komponenten und Plasmakomponenten umfasst. An zellulären Komponenten sind neben den Erythrozyten Leukozyten enthalten, u.a. Lymphozyten (B- und T-Lymphozyten, NK-Zellen, NK/T-Zellen), Thrombozyten, Granulozyten (auch als polymorphkernige Leukozyten bezeichnet: Neutrophile, Eosinophile, Basophile), Monozyten/Makrophagen, dendritische Zellen, Mastzellen und ihre Vorläufer (z.B. hämatopoetische Stammzellen) . An Plasmakomponenten sind z.B. Komplementsystem, Gerinnungsfaktoren, Botenstoffe wie Cytokine und Hormone oder Salze zu nennen. Die Zusammensetzung von Blut Gesunder ist z.B. in "Labor und Diagnose" beschrieben (Thomas 2000).

Viele Erkrankungen gehen mit z.T. komplexen Veränderungen der Blutkomposition einher, wodurch pathophysiologisch relevante Prozesse initiiert bzw. aufrechterhalten werden. Medikamentöse Korrekturversuche bleiben oft auf die Neutralisierung bzw. Normalisierung von Einzelfaktoren beschränkt und können daher häufig nicht den gewünschten klinischen Normalisierungseffekt erreichen. Gleiches gilt für die Transfusion von Vollblut bzw. Blutbestandteilen sowie für herkömmliche extrakorporale Blutbehandlungsverfahren, die in der Regel lediglich eine grobe Änderung der Zusammensetzung des Blutes bewirken, wie etwa die Entfernung einer Gesamtfraktion des Blutes, wobei neben entfernenswürdigen Substanzen (z.B. Toxinen) auch wertvolle Substanzen verloren gehen (z.B. beim Plasmaaustausch). Andere extrakorporale Verfahren wiederum zielen auf selektive Entfernung von Einzelschadstoffen und wirken daher nur punktuell (z.B. spezifische Adsorption).

### Medikamentöse Therapien

Medikamentöse Therapieansätze stellen eine wesentliche Säule in der modernen Medizin dar. In der Mehrzahl der Fälle wirkt das Medikament dabei systemisch. Die systemisch wirkenden Mittel betreffen das Blut insofern, als dass der Medikamenteneffekt in der Regel über das Blut vermittelt und die Blutkomposition und/oder -funktion beeinflusst wird. Diese Wirkung ist häufig punktuell, d.h. ein Medikament behebt ein singuläres Problem, etwa beim Ausgleich von Mangelzuständen (Elektrolytgabe bei Elektrolytmangel, Glucosegabe bei Glucosemangel, Hormonsubstitution bei entsprechendem Mangel usw.) oder löst einen Mechanismus aus, der hilft, ein pathophysiologisches Problem gegenzuregulieren (z.B. Antikoagulation bei Hyperkoagulabilität und Gefäßwandschäden, Immunsuppression bei Autoimmunprozessen, Gabe von neutralisierenden Antikörpern bei Immundysfunktionen).

Ein weiteres Anwendungsgebiet medikamentöser Therapien stellt die - mehr oder weniger selektive - Abtötung von schädlichen körpereigenen Zellen (Chemotherapie bei Tumoren) oder körperfremden Organismen (Gabe von Antibiotika bei Infektionen) dar. Die Blutkomposition und -funktion kann auch durch Blutzell-Wachstumsfaktoren (Erythropoietin, Thrombopoietin, G-CSF, GM-CSF) auf der Zellseite und durch Vitamin K-Gaben auf der Plasma-Seite verändert werden.

Diese punktuellen Therapien können oft jedoch alleine nicht das angestrebte Ziel erreichen, sondern bedürfen dazu z.B. noch der Mitwirkung körpereigener Systeme. Auch der Einsatz neutralisierender Antikörper mit dem Ziel der Korrektur pathologischer Toxin- bzw. Botenstoff-Konzentrationen, z.B. Anti-LPS, Anti-TNF alpha etc. ist oft nicht ausreichend zur Heilung einer Krankheit. Ein weiterer Nachteil der medikamentösen Therapien sind die unerwünschten Arzneimittelwirkungen, die z.T. gravierend sein können und im ungünstigsten Fall den Krankheitsverlauf verschlechtern bzw. sogar zum Tode des Patienten führen können (z.B. allergische und toxische Reaktionen, opportunistische Infektionen nach Chemotherapie oder Immunsuppression, unerwünschte Effekte der Aktivierung von Immunzellen).

### Blut-/Blutzell-/Plasma-Transfusionen

Blut- und Plasma-Transfusionen gehören zur Standardtherapie der modernen Medizin. Sie werden gezielt zum Ausgleich von Mangelzuständen im Patientenblut (Zytopenien, Volumenmangel, Gerinnungsfaktorenmangel u.a.) eingesetzt. Neben Vollbluttransfusionen werden vor allem Konzentrate der einzelnen Blutzellarten eingesetzt (Erythrozyten-, Thrombozyten-, Granulozytenkonzentrate). Während Erythrozyten bei Anämie und Thrombozyten bei Thrombopenie mit Blutungsgefahr gegeben werden, ist das Einsatzgebiet von Granulozytenspenden der Granulozytenmangel (im Extremfall Agranulozytose) zur Behandlung neutropener Infektionen. Durch die Einführung von Steroid- bzw. G-CSF-induzierten Granulozytenkonzentraten mit hoher Zellzahl wurden die klinischen Ergebnisse bei Granulozytentransfusion verbessert (Stanworth 2005, Safdar 2004).

Es sind Verfahren zur Behandlung von hepatischem Koma bekannt, bei denen das Blut des Patienten vollständig aus dem Körper des Patienten ausgewaschen und mit Ringer Laktat-Lösung, z.T. ergänzt mit Albumin, ersetzt wird (total body washout), und der Kreislauf anschließend wieder mit normalem Blut gefüllt wird. Verfahren, in denen das Blut eines Patienten vollständig durch Bluttransfusion ersetzt wird, werden seit längerer Zeit eingesetzt.

Die Vorteile sind Entfernung von Giften aus dem Kreislauf und die Zufuhr von frischen/gesunden Zellen und Plasmakomponenten wie Koagulationsfaktoren. Direkte Transfusion von Spender zu Empfänger führte zu den besten Ergebnissen, ist jedoch unpraktikabel, da mehrere kompatible Spender für jede Behandlung anwesend sein müßten. Daher muß mit Zitrat oder Heparin behandeltes Blut verwendet werden, was zu Azidose, Hyperkaliämie, hohem Citratspiegel und Hyperammoniämie sowie Störungen des Säure-Base-Gleichgewichtes im Blut führen kann. In späteren Leberversagens-Studien wurde eine schlechte Überlebensrate festgestellt. Extrahepatische Komplikationen bei dem Verfahren schließen Aspiration, Blutungen, Hypoglykämie, Sepsis, Bronchospasmen und Gehirnödem ein, oft mit Todesfolge.

Bei Patienten mit Neutropenie, neutrophiler Dysfunktion, mit Krebs-assoziierten Infektionen oder Sepsis führten Granulozytentransfusionen (GTx), insbesondere mit hoher Dosis, z.T. zu einer Verbesserung der Überlebensrate (Safdar 2004, Stanworth 2005). Allogene Bluttransfusionen erhöhen jedoch das Risiko von multiplem Organversagen. Die Transfusion von Leukozyten bringt auch ein immunsuppressives Potential mit sich, dass die klinische Situation von Sepsis-Patienten verschlechtern kann (Moore 1997, Gianotti 1993).

### Extrakorporale Verfahren

Als extrakorporale Behandlungsverfahren sind Dialyse / Filtration, Plasmaaustausch / Plasmaperfusion / Plasmaadsorption, Bioreaktor-Therapien oder extrakorporale Organperfusion zu nennen.

Für einen Überblick über extrakorporale Blut-Behandlungsverfahren bei unterschiedlichen Indikationen wird auf die folgenden Veröffentlichungen verwiesen: *Akute Nierenversagen und Blutvergiftungen* (Ronco 1998), *Leberversagen* (van de Kerkhove 2004, Mitzner 2007), Neurologische *Autoimmunerkrankungen* (Lehmann 2006), *Fettstoffwechsel-Erkrankungen* (Blessing 2004), *Sepsis* (Bellomo 2005).

Extrakorporale Blutentgifungsverfahren können z.B. bei Sepsis und mehrfachem Organversagen den klinischen Verlauf entscheidend beeinflussen (Ronco 2003). Hämofiltration, Hämodialyse, Plasma- oder Vollblutperfusion durch adsorbierende Säulen (e.g. Aktivkohle, immobilisiertes Polymyxin B oder DEAE) wurden in diesem Zusammenhang verwendet (Bellomo 2005). Cytokine können durch Hämofiltration/Plasmafiltration in einem wesentlichen Ausmaß aus dem Kreislauf von septischen Patienten entfernt werden, was jedoch bei einigen Studien nicht unbedingt zu einer signifikanten Steigerung der Überlebensrate führte (Cole 2002). Größere kontrollierte Studien fehlen noch, die vorhandenen Daten lassen jedoch darauf schließen, dass solche Verfahren, die größere Moleküle/ Partikel aus Plasma entfernen können (z.B. Behandlung mit großen Volumina, Filtration mit großen Poren, Plasmapherese und Adsorption) einen größeren Effekt auf den Krankheitsverlauf haben als solche, die vor allem kleinere wasserlösliche Moleküle betreffen (Busund 2002, Stegmayr 2003, Ronco 2002, Tetta 2003).

Es wurde gezeigt, das Hämoperfusion über Aktivkohle positive Effekte bei externer Vergiftung hat (Vale 1975). Bei hepatischem Koma aus verschiedenen Ursachen wurde das Verfahren seit 1972 mit wechselndem Erfolg eingesetzt (O'Grady 1988). Ionenaustauschharze können insbesondere Protein-gebundene Substanzen binden und werden daher ebenfalls z.B. zur Behandlung von Leberversagen eingesetzt (Weston 1974).

Die wichtigsten Nachteile der Hämoperfusion sind der Mangel an Selektivität, da auch eine Adsorption von Hormonen, Vitaminen, Immunglobulinen, Medikamenten und andern Plasmakomponenten auftritt, sowie eine Aktivierung der Komplementkaskade, Thrombozytopenie and Leukozytenmarginalisierung.

Hämodialyse, vor allem mit großporigen Membranen, z.B. der Hochdurchflußmembran AN69 (polyacrylonitrile, PAN), und einem geschlossenen Dialysatkreislauf mit sterilem mit Acetat gepuffertem Dialysat, wurde ebenfalls zur Behandlung von Leberversagen eingesetzt (Opolon 1976, Konstantin 1992). Bei diesem Verfahren können auch mittelgroße Moleküle mit einem Molekulargewicht von bis zu 5000 Dalton entfernt werden, nicht aber hydrophobe und/oder Protein-gebundene Substanzen wie kurz- oder mittelkettige Fettsäuren, Merkaptane oder Bilirubin.

Hämodiasorption ist ein Prozeß, bei dem Blut durch Packungen von Dialysemembranen, umgeben durch eine Suspension von feinen absorbierenden Partikeln, strömt und stellt damit im wesentlichen eine Kombination eines Siebverfahrens (Dialyse) und einer Absorption dar. Dabei wurde (Ash 1994) z.B. ein Dialysesystem mit einer absorbierenden Suspension genutzt, bei dem ein Dialysegerät mit flacher Zellulosemembran auf einer Seite mit dem Blut des Patienten und auf der anderen Seite mit einem Dialysat aus einer Mischung von Aktivkohle und Anionaustauscherharz-Partikeln perfundiert wurde.

Dialysesysteme mit Hohlfasermembranen und 20 %igen humanem Serumalbumin als molekularem Adsorptionsmittel (molecular adsorbent recirculating system, MARS) (Mitzner 2007) sind ebenfalls zur Behandlung von Blutkompositionstörungen wie bei Leberversagen, bekannt.

Bei therapeutischem Plasmaaustausch (therapeutic plasma exchange (TPE)) wird das Blut des Patienten entweder zentrifugiert oder filtriert, um die Blutzellen vom Plasma zu trennen. Das Plasma des Patienten wird dann durch das Plasma gesunder Spender ersetzt, normalerweise frisches gefrorenes Plasma (fresh frozen plasma, FFP), und dieses mit den Blutzellen dem Patienten transfundiert. FFP ist leichter erhältlich und handhabbar als Vollblut, was einen Vorteil gegenüber Austausch von vollständigem Blut ausmacht. Ein Überblick wird in (Bambauer 1988) gegeben. Es gibt jedoch eine hohe Komplikationsrate, besondere bakterielle Infektionen, Lungenödem und Blutungen. Bei Verwendung von Citratplasma treten trotz zusätzlicher Dialyse und Substitution von Calcium hypocalcemische Krämpfe auf.

Die therapeutische Verwendung von extrakorporalen Bioreaktoren hat bereits lange Tradition. Konzepte basierend auf Zell-basierter extrakorporaler Organunterstützung wurden erfolgreich bei aktutem Leberversagen (Allen 2001, Demetriou 2004) und akutem Nierenversagen assoziiert mit Sepsis (Humes 2004) verwendet.

Im Stand der Technik wird eine extrakorporale Plasmaperfusion mit Einsatz eines Bioreaktors mit immunmodulatorischen Zellen (wie Endothelzellen, Leukozyten, von hämotopoietischen Stammzellen durch Differenzierung abgeleiteten Zellen oder Linien dieser Zelltypen), welche immunmodulatorische Stoffe über Rezeptoren adsorbieren oder solche Stoffe freisetzen können, vorgeschlagen (DE 198 31 873, WO 01/51068). Auch eine extrakorporale Plasmaperfusion eines Bioreaktors mit Phagocyten wie Granulozyten oder Monozyten/Makrophagen wird vorgeschlagen, in dem Funktionen der Granulozyten genutzt werden sollen, um das Plasma des Patienten durch Phagozytose von Schadstoffen und Pathogenen zu reinigen. Eine Ergänzung der Zellen im Bioreaktor durch einzelne andere Zellen, wie Hepatozyten oder Endothelzellen wird erwähnt. Es wird z.B. vorgeschlagen, Zelllinien oder aus hämatopoietischen Stammzellen differenzierte Zellen einzusetzen.

Dem gegenüber stellt sich dem Fachmann das Problem, ein Therapieverfahren bzw. Therapiesystem zur Verfügung zu stellen, das die momentane Patientensituation biosensorisch analysieren kann und gleichzeitig individuell, spezifisch, aber komplex und umfassend die Blutkomposition regulierend verändern kann.

Das Problem wird durch den Gegenstand der Ansprüche gelöst. Insbesondere wird ein Verfahren zur Behandlung von Blutkompositionsstörungen und Blutfunktionsstörungen zur Verfügung gestellt, das Schritte umfasst, bei denen man Blut oder Blutplasma eines Patienten extrakorporal mit einer zweiten Flüssigkeit (Bioäquivalent) in Kontakt bringt, wobei die zweite Flüssigkeit Blut oder modifiziertes Blut ist, welches Granulozyten, Thrombozyten und Erythrocyten umfasst.

Insbesondere umfasst das Bioäquivalent mindestens 0,4 x 10¹⁰/l Leukozyten, insbesondere 1-200 x 10¹⁰/1 Leukozyten, davon mindestens 0,2 x 10¹⁰/1 Granulozyten, mindestens 1,0 x 10⁹/1 Thrombozyten und mindestens 1,0 x 10⁹/l Erythrozyten.

Bevorzugt umfasst das Bioäquivalent insbesondere 1-100 x 10¹⁰/l Granulozyten, bevorzugt mindestens 1,5 x 10¹⁰/1 Granulozyten oder mindestens 3 x 10¹⁰/1 Granulozyten. Bevorzugt umfasst das Bioäquivalent 1,0 x 10⁹/l - 5 x 10¹¹/l Thrombozyten, insbesondere mindestens 5 x 10⁹/1 oder mindestens 1 x 10¹⁰/1 Thrombozyten. In einer bevorzugten Ausführungsform umfasst das Bioäquivalent mindestens 1,0 x 10⁹/l Erythrozyten, insbesondere 5,0 x 10⁹/1 - 8,0 x 10¹²/1 Erythrozyten oder 0,1- 8,0 x 10¹²/l Erythrozyten, typischerweise aber 0,5-6,0 x 10¹²/1 Erythrozyten. Derartige Werte werden wegen der großen Zahl an Erythrozyten in Blut auch bei einer recht starken Abreicherung an Erythrozyten erreicht.

Es wurde im Rahmen der Erfindung festgestellt, dass die Verwendung von Blut oder modifiziertem Blut, das insbesondere die Zelltypen der Granulozyten und Thrombozyten in großer Konzentration umfasst, sich überraschend positiv auf das klinische Ergebnis auswirkt. Durch die Nutzung von modifiziertem Blut anstelle von einzelnen Zelltypen wie im Stand der Technik wird sichergestellt, dass das Bioäquivalent dazu in der Lage ist, alle wesentlichen Organfunktionen von gesundem Blut zur Behandlung der Kompositionsstörungen und/oder Funktionsstörungen des Patientenbluts bereitzustellen.

Besonders bevorzugt ist es daher, dass das Bioäquivalent auch signifikante Mengen an Lymphozyten und/oder Monozyten (Monocyten/ Makrophagen) umfasst. Es sollten alle Zelltypen, die in gesundem Blut vorkommen, vertreten sein, die Konzentrationen können jedoch von den normalen Konzentrationen in Blut abweichen. In einer bevorzugten Ausführungsform umfasst das Bioäquivalent mindestens 0,4 x 10¹⁰/1, mehr bevorzugt 2,0 x 10¹⁰/1 Lymphozyten und insbesondere mindestens 1,0 x 10⁸/1 Lymphozyten. In einer bevorzugten Ausführungsform umfasst das Bioäquivalent mindestens 1,0 x 10⁸/1 Monozyten.

Es wird bevorzugt ein Volumen des Bioäquivalents von ca. 100mL bis ca. 20L, insbesondere ca. 200 mL, ca. 500 mL, ca. 1 L, ca. 2 L oder ca. 5 L eingesetzt.

In einer Ausführungsform der Erfindung umfasst das Bioäquivalent ferner Blutplasma. Dieses kann wesentliche Faktoren zur Behandlung von Funktionsstörungen und Kompositionsstörungen beitragen, indem z.B. fehlende Plasmafaktoren des Patientenbluts supplementiert werden. Auch eine Ausverdünnung von wichtigen Plasmafaktoren des Patientenbluts wird verhindert. Alternativ kann das Blutplasma in dem modifizierten Blut durch physiologische Kochsalzlösung oder einen biokompatiblen Puffer teilweise oder ganz ersetzt sein. Z.B. kann die selektive Abreicherung einzelner Plasmafaktoren in bestimmten Krankheitssituationen sinnvoll sein. Dies wird von der Situation des Patienten und eventuellen Inkompatibilitäten von Spenderblut und Patientenblut abhängen.

Als Bioäquivalent wird im Rahmen der Erfindung Blut oder modifiziertes Blut eines gesunden Spenders bezeichnet, das im Rahmen einer extrakorporalen Behandlung von Blut oder Blutplasma eines Patienten Organfunktionen dieses Patienten übernehmen und/oder ergänzen kann, im Sinne einer temporären auxilliären Organtransplantation. Das durch die Erfindung zur Verfügung gestellte Bioäquivalent behält wesentliche, sich aus der Komplexität des Vollblutes als Organ ergebende Eigenschaften und ist dem Vollblut - teilweise oder komplett - funktionell identisch. Insbesondere soll das Bioäquivalent dem Vollblut ähnliche biospezifische Adsorption bzw. Neutralisation ermöglichen. Eine Modifikation des Vollblutes sollte diese Eigenschaft so wenig wie möglich, bevorzugt nicht, beeinflussen. Durch die Modifikation werden bevorzugt günstige, erwünschte Eigenschaften verstärkt (z.B. durch Erhöhung des Aktivitätszustandes oder der Konzentration einzelner Plasmabestandteile oder Zelltypen), ungünstige, nicht erwünschte Eigenschaften werden abschwächt (z.B. durch Abreicherung einzelner Plasmabestandteile oder Zelltypen, Volumenmodifikation). Vollblut selbst ist ein Bioäquivalent im Sinne dieser Definition, bei dem der Grad der Modifikation null ist. Eine typische Quelle für Bioäquivalente sind humane Blutspenden. Die Modifikation kann u.a. durch Zusätze, Passage über Adsorbenzien, Filtrations- und Trennprozesse sowie physikalische oder chemische Prozessierung erfolgen.

Das Bioäquivalent kann Blut umfassen, das z.B. durch Volumeneinengung (z.B. durch anteiligen Plasmaentzug), Leukozytenfraktionsanreicherung (z.B. durch geeignete Zentrifugationsschritte), Leukozytendepletion (oder Depletion von einzelnen Leukozytentypen, z.B. von B-Lymphozyten), Erythrozytendepletion, Depletion von Antikörpern, Bestrahlung, Gerinnungshemmung und/oder Stabilisierung modifiziert ist.

Eine Depletion von Antikörpern kann z.B. vorteilhaft sein, um Nebenwirkungen wie transfusionsassoziiertes Lungenversagen zu verhindern. Sie kann z.B. über Adsorption von Antikörpern an einer Protein A oder Protein G-Säule erreicht werden.

Um eine Lagerung des Bluts zu vereinfachen, können Stabilisatoren (z.B. Puffer oder Zell-Nährlösungen) und gerinnungshemmende Substanzen (z.B. Citrat-basierte Lösungen wie etwa ACD-Lösung (Acid-Citrate-Dextrose, eine Lösung von Zitronensäure, Natriumcitrat und D-Glucose in Wasser) hinzugefügt werden. Zur Behandlung einiger Erkrankungen, z.B. bei Sepsis, kann es auch vorteilhaft sein, das modifizierte Blut zu konditionieren, also z.B. mit LPS vorzustimulieren.

Bevorzugt sind in dem modifizierten Blut Granulozyten und Thrombozyten angereichert. Das Bioäquivalent ist etwa durch Plasmapherese erhältlich, wobei z.B. die Granulozytenbildung durch vorherige Gabe von Stimulatien wie G-CSF oder Steroiden gefördert worden sein kann. Das modifizierte Blut kann ein Granulozytenkonzentrat oder ein Buffy Coat sein. Verfahren zur Gewinnung eines Granulozytenkonzentrats oder eines Buffycoats sind im Stand der Technik bekannt.

Das im Rahmen der Erfindung eingesetzte Blut oder modifizierte Blut ist bevorzugt nach den Kriterien für eine Bluttransfusion ausgesucht (z.B. blutgruppengleich) und/oder überwacht. Der Spender sollte gesund sein. Blutspenden werden üblicherweise auf die Anwesenheit von Krankheitserregern getestet, was auch vorliegend Anwendung finden sollte. Normalerweise wird das Verfahren bei Menschen angewandt, d.h., sowohl Patient als auch Empfänger sind Menschen, grundsätzlich ist jedoch auch eine Nutzung bei Tieren möglich.

Durch den Einsatz von gesundem Vollblut bzw. eines geeigneten bioäquivalenten Auszugs (Bioäquivalent) in einem mit dem Patientenblut oder Blutplasma kommunizierenden, extrakorporalen System zur Behandlung von Blutfunktions- und Blutkompositionsstörungen wird das effektive Blut- bzw. Plasmavolumen des Patienten derart erweitert, dass zirkulierende Toxine (a) unter eine kritische Grenze ausverdünnt bzw. (b) biospezifisch im Bioäquivalent adsorbiert/neutralisiert werden. Das Bioäquivalent kann darüber hinaus (c) als Gesamtorgan aktiv, umfassend und komplex auf Schadreize reagieren. Es findet eine komplexe Biokommunikation zwischen Vollblut/Bioäquivalent einerseits und Patientenblut andererseits statt. Im Rahmen dieses biochemischen Informationsaustausches (I) "lernt" das Bioäquivalent über den Krankheitszustand des Patienten und (II) überwindet das Bioäquivalent die Kommunikationsstarre bzw. reguliert die Fehlkommunikation im Patientenblut. Es erfolgt eine Normalisierung auch komplexer Schadstoffmuster bzw. geeignete Bioprozessierung, wodurch ein Milieu entsteht, dass organregenerative Prozesse ermöglicht und in dem sich der Gesundheitszustand des Patienten stabilisiert.

Unter komplexer Biokommunikation wird die Gesamtheit des im und mit Bioäquivalent stattfindenden biochemischen, physikalischen und sonstigen Signalaustausches verstanden. Dazu zählen Zell-Zell-Kommunikation (durch direkten Kontakt, z.B. über Rezeptoren und indirekt über Botenstoffe), Zell-Plasma-Kommunikation und Plasma-Plasma-Kommunikation. Störungen der Komposition des Bioäquivalents durch Kontakt mit dem Blut oder Plasma des Patienten wird in der Regel eine modifizierte komplexe Biokommunikation nach sich ziehen. Dies kann gezielte Antwort-Reaktionen und Prozesse initiieren, die zu einer Gegenregulation führen.

Bei dem erfindungsgemäßen Verfahren sollen die Zellen des Bioäquivalents nicht dem Körper des Patienten zugeführt werden. Dies kann in einer Ausführungsform erreicht werden, indem man das erfindungsgemäße Verfahren so durchführt, dass es Schritte umfasst, bei denen man
a) aus Patientenblut gewonnenes Blutplasma (gleichzeitig zu dessen Gewinnung oder zeitverzögert) dem Bioäquivalent zuleitet,
b) durch einen Filtrationsschritt das modifizierte Blutplasma von den Zellen des Bioäquivalents trennt, damit es dem Patientenblut wieder zugeführt werden kann.

Dabei kann das Blutplasma aus Patientenblut z.B. durch Anlegen eines Druckgradienten über eine semipermeable Membran gewonnen wurde. Eine Gewinnung ist ebenfalls über Zentrifugation oder Plasmapherese möglich. Geeignete Verfahren und Vorrichtungen sind im Stand der Technik bekannt. Die bevorzugte Variante ist die Membran-Plasmaseparation unter Nutzung eines Einmal-Gebrauchs-Hohlfaser-Plasmaseparators (z.B. unter Verwendung des Gerätes BM25 von Baxter/Edwards und eines Membran-Plasmafilter PF 1000 von Gambro AB, Lund).

In einer anderen Ausführungsform der Erfindung werden Patientenblut oder Patientenblutplasma mit dem Bioäquivalent in Kontakt gebracht, wobei Patientenblut oder Patientenblutplasma und das Bioäquivalent durch eine semipermeable, plasmadurchlässige Membran voneinander getrennt sind.

Bevorzugt liegt die Ausschlussgrenze der Membranporen einer im Rahmen der Erfindung verwendeten semipermeablen Membran, die für Plasma durchlässig ist, jedoch nicht für Zellen, zwischen minimal etwa 5.000 Dalton und maximal bei einem durchschnittlichen Porendurchmesser von ca. 0,8 µm. Ober die Membraneigenschaften (durchschnittliche Porengröße, Polymermaterialien, Membranarchitektur) kann regulierend auf den transmembranalen Stoff- und Informationsaustausch Einfluß genommen werden. Eine typische Membran hat einen durchschnittlichen Porendurchmesser von ca. 0,2 µm.

Die Membran kann z.B. eine Hohlfasermembran oder eine Flachmembran sein. Die Membran kann eine auf einer oder beiden Seiten beschichtete oder unbeschichtete Membran sein. Materialen aus denen die Membran bestehen kann sind z.B. Polycarbonat, PE, PTFE, Polypropylen oder Nylon. Eine bevorzugter Membran-Plasmaseparator besteht z.B. aus Polypropylen-Hohlfäden und hat eine effektive blutseitige Membranoberfläche zwischen 0,1 und 0,5 m².

Da in dem erfindungsgemäßen System - im Unterschied zu herkömmlichen Dialysesystemen - auf der Dialysatseite Blut oder ein Bioäquivalent verwendet werden, ist es bevorzugt, dass die Membran mindestens auf dieser Seite biokompatibel (d.h. für die dort befindlichen Blutzellen nicht toxisch ist bzw. diese nicht aktiviert) ist, bevorzugt ist sie dies auf beiden Seiten. Dazu können Beschichtungen der Membran beitragen, z.B. Beschichtungen aus biologisch aktiven Substanzen (wie Gerinnunghemmern, Proteinen) oder bio-inerten Substanzen (wie Metalle oder Keramikwerkstoffe).

Da die Zellen des Bioäquivalents dem Patienten nicht zugeführt werden, ist eine Bestrahlung dieser Zellen zwar möglich, aber nicht unbedingt erforderlich.

Bevorzugt wird das Patientenblut oder Patientenblutplasma in einem extrakorporalen Kreislauf gerichtet bewegt und/oder das Bioäquivalent wird gerichtet bewegt. Dabei ist sowohl eine gegenläufige als auch eine gleichgerichtete Bewegung möglich, d.h. die beiden Flüssigkeiten werden entweder in entgegengesetzen Richtungen entlang der Trennmembran geführt (countercurrent flow) oder im Parallel-Fluss. Unter allen Bedingungen sollte gewährleistet sein, dass das in Kontakt bringen von Patientenblut oder Patientenblutplasma unter geeigneten Bedingungen (ähnlich wie für eine Zellkultur) und für eine geeignete Zeit erfolgt, um die Verbesserung der Blutfunktionsstörungen und Blutkompositionsstörungen nach Zufuhr des behandelten Patientenbluts/Patientenblutplasmas zu erreichen.

Es ist anzumerken, dass das erfindungsgemäße Verfahren sowohl durchgeführt werden kann, wenn das Patientenblut oder Patientenblutplasma in einem extrakorporalen Kreislauf mit dem Kreislauf des Patienten verbunden oder von diesem getrennt ist, etwa nach Gewinnung von Patientenblutplasma z.B. durch Plasmapherese, Inkubation des Plasmas mit dem Bioäquivalent, Abtrennung von mindestens der Zellkomponenten des Bioäquivalents vor Infusion beim Patienten.

Die Ausführungsform der Erfindung, in der Patientenblut oder Patientenblutplasma und das Bioäquivalent durch eine semipermeable, plasmadurchlässige Membran voneinander getrennt sind, löst das Problem, Patientenblut so in Kontakt mit dem Bioäquivalent zu setzen, dass einerseits eine komplexe Biokommunikation zwischen gesundem und kranken Blut möglich wird und andererseits eine Kontrolle über den Austausch und bevorzugt auch eine jederzeit mögliche Kompletttrennung der Systeme realisiert werden kann, wobei dies Volumenbilanzneutral, also ohne Störung der Volumenhomöostase des Patienten erfolgen kann.

Gleichzeitig stellt die Membran eine Sicherheitsbarriere für den Patienten dar (etwa gegen zellvermittelte allergische Reaktionen, Infektionen, Zellübertritte mit nachfolgender Induktion von Organschäden, z.B. akuter Lungenschädigung, etc.).

Als Bioäquivalenzraum wird im Rahmen der Erfindung der extrakorporal gelegener Raum bezeichnet, in dem sich das Bioäquivalent während der Behandlung befindet. Der Bioäquivalenzraum ist vom Patientenblut in einer Ausführungsform durch eine semipermeable Membran getrennt. Durch die Membran kommunizieren Patientenblut(plasma) und Bioäquivalent miteinander. Die Verbindung von Bioäquivalenzraum und Patientenblut(plasma) ist reversibel und kann jederzeit gelöst werden.

Es können dadurch bevorzugt die folgenden potentiell unerwünschten Resultate der Kommunikation reguliert/unterbunden werden:
a) Entwicklung bzw. Verstärkung von ungünstigen, potenziell patientenschädlichen Eigenschaften des Bioäquivalentes,
b) Zelldebris-Einstrom aus dem Bioäquivalenzraum in den Patienten,
c) Einstrom von schädlichen Zellprodukten/Plasmakomponenten in den Patienten,
d) Verlust von nützlichen Zellprodukten/Plasmakomponenten aus dem Patienten.
e) Hämodynamische Instabilität des Patienten durch Volumenverluste.

Um auf diese Parameter von außen Einfluß nehmen zu können, ist es hilfreich, wenn eine Zustandserfassung des Bioäquivalentes und/oder des Patientenbluts/Patientenblutplamas z.B. durch laborchemische oder physikalische Methoden erfolgt. Diese kann laufend erfolgen und ist bei allen Ausführungsformen der Erfindung sinnvoll.

Bei dem erfindungsgemäßen Verfahren handelt es sich in einer Ausführungsform um ein extrakorporales Blutbehandlungs-Verfahren, welches am Patientenbett betrieben werden kann, zur Korrektur von komplexen Schadstoffmustern durch
a) Abreicherung von Schadstoffen/Einflussminderung von Schadprinzipien mittels Ausverdünnung und/oder
b) biospezifische Adsorption/Neutralisation im Bioäquivalenzraum und/oder
c) bioaktive Gegenregulation, die durch das Bioäquivalent vermittelt durch
d) komplexe biosensorische Online-Analyse zwischen Patientenblut und dem therapeutischen Bioäquivalent
e) transmembranale gesteuerte komplexe Biokommunikation

im membranseparierten Zwei-Kompartmentsystem (extrakorporaler Blutkreislauf und Bioäquivalenzraum.

Das erfindungsgemäße Verfahren wird auch als Bioäquivalenzdialyse (bioequivalence dialysis, BED), Dysäquilibrium-Vollblut-Dialyse (DVD) oder Biosystem-Dialyse (BSD) bezeichnet.

Im Rahmen der vorliegenden Erfindung kann eine Blutkompositionsstörung oder Blutfunktionsstörung z.B. Sepsis oder septischer Schock, insbesondere in der Phase der Immunparalyse, Niereninsuffizienz, Leberinsuffizienz, Immundefizienz oder eine Infektionskrankheit sein oder damit in Verbindung stehen. Ein Einsatzgebiet des erfindungsgemäßen Verfahrens ist damit z.B. die Therapie der Sepsis. In fortgeschrittenen Sepsisstadien (schwere Sepsis und septischer Schock) liegt eine komplexe Blutkompositions- und -funktionsstörung vor. Ein zentrales Element dieser Störung ist die Immunparalyse (auch Compensatory Antiinflammatory Response Syndrome, CARS)(Oberholzer 2001). Es wurde gezeigt, dass funktionelle Beeinträchtigung der Neutrophilen oder Monozyten als zentraler Komponenten des angeborenen Immunsystems klar mit einer gesteigerten Mortalität in fortgeschrittenen Stadien von Sepsis und septischem Schock assoziiert sind (Docke 1997, Caille 2004, Ploder 2006, Kaufmann 2006).

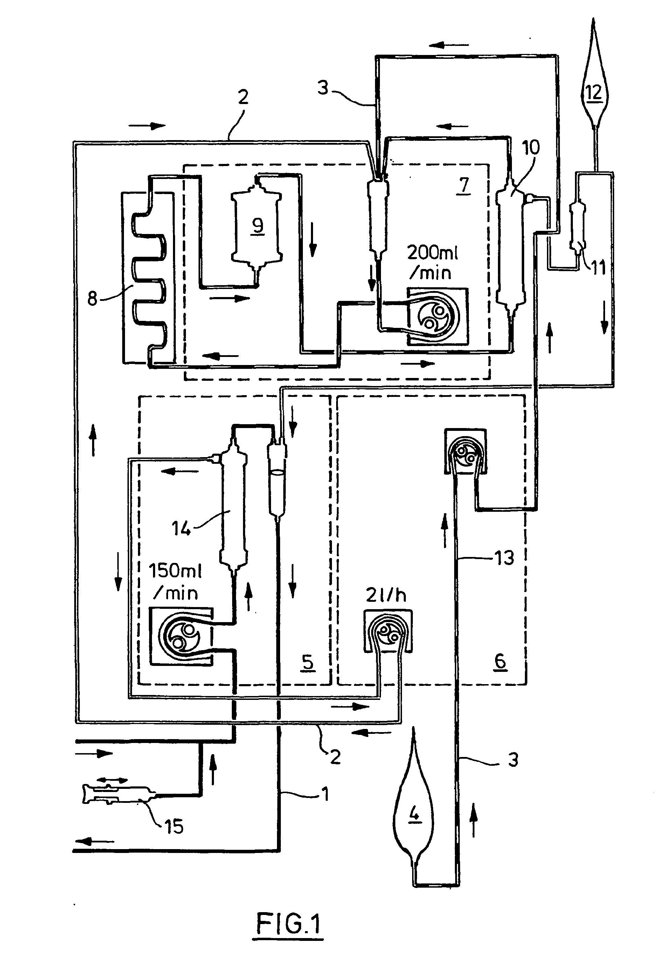

Im Rahmen der vorliegenden Erfindung wird auch eine Vorrichtung zur Durchführung des erfindungsgemäßen Verfahrens zur Verfügung gestellt, welche zwei durch eine oder mehrere semipermeable Membranen getrennte Räume umfasst, von denen einer zur Aufnahme von Patientenblut oder Blutplasma geeignet ist und der zweite zur Aufnahme des Bioäquivalents geeignet ist. Diese Eignung ergibt sich insbesondere daraus, dass die Membran, wie oben geschildert, biokompatibel ist, vor allem (auch) die Membranseite, welche mit dem Bioäquivalent in Kontakt steht. In einer Ausführungsform der Erfindung ist die Vorrichtung eine herkömmliche Dialysevorrichtung, welche mit einer semipermeablen Membran ausgerüstet ist, die mindestens auf der Seite, die mit dem Bioäquivalent in Kontakt steht, biokompatibel ist. Die Vorrichtung kann bereits Bioäquivalent in dem zweiten Raum (dem Bioäquivalenzraum) umfassen. Die Vorrichtung ist bevorzugt eine Vorrichtung, wie sie in Fig. 1 gezeigt ist und umfasst die dort schematisch dargestellten Bestandteile. Insbesondere kann die erfindungsgemäße Vorrichtung einen MARS-Monitor (Gambro AB) und je ein BM11 und BM14-Gerät (Baxter/Edwards) umfassen. In einer besonderen Ausführungsform ist das Volumen des zweiten Raums variabel, es liegt bevorzugt zwischen 100mL und 20L.

Die Erfindung lehrt auch die Verwendung einer erfindungsgemäßen Vorrichtung zur Behandlung einer Blutkompositionsstörung oder Blutfunktionsstörung.

Ein weiterer Gegenstand der Erfindung ist die Verwendung von Blut oder einer daraus gewonnenen Flüssigkeit, welche Granulozyten, Thrombozyten und Erythrozyten umfasst und deren bevorzugte Ausgestaltungen oben beschrieben sind (Bioäquivalent), zur Behandlung einer Blutkompositionsstörung oder Blutfunktionsstörung, oder zur Herstellung eines Medikamentes oder Medizinprodukts zur Behandlung einer Blutkompositionsstörung oder Blutfunktionsstörung. Bei dieser Behandlung werden, wie oben beschrieben, Blut oder Blutplasma eines Patienten extrakorporal mit dem Bioäquivalent in Kontakt gebracht.

Im Rahmen der vorliegenden Erfindung wird erstmals beschrieben, wie eine effektive Erweiterung des Blutorganraums vorgenommen wird, ohne mögliche Nebenwirkungen wie Allergien oder zellvermittelte Infektionen und Gewebeschädigungen akzeptieren zu müssen. Erstmals wird dabei die Funktion des Blutes als komplexes Flüssigorgan therapeutisch extrakorporal und reversibel eingesetzt. Eine Glättung von komplexen pathologischen Zustandsmustern im Patientenblut wird erreicht. Medizinisch liegt der Vorteil in einer qualitativ und quantitativ neuartigen Dimension der Patientenblut-Zustands-Änderung, die sich klinisch durch - gegenüber dem herkömmlichen Stand der Technik - bessere klinische Ergebnisse bemerkbar macht.

Vom Stand der Technik unterscheidet sich die erfindungsgemäße Lehre auch dadurch, dass nicht induktiv vorgegangen wird, sondern deduktiv. Das heißt, es werden keine Einzellösungs-Bausteine für Einzelprobleme definiert, oder es soll keine willkürlich konstruierte Kombination von Einzelbausteinen zu einer Systemlösung führen (wie etwa bei Leberzell-Bioreaktoren oder dem in den Anmeldungen DE 195 19065, DE 198 31 873 oder WO 01/51068 vorgeschlagenen System), sondern ein allgemein bekanntes, biologisch als Funktionseinheit definiertes, komplettes Biosystem (Blut) kommt entweder unverändert oder vom allgemeinen (normales Vollblut) auf das Besondere hin verändert zum Einsatz. Unter "Veränderung auf das Besondere hin" soll verstanden werden, dass Vollblut auf die spezifischen Anforderungen der späteren Behandlung vorbereitet wird, wie dies oben geschildert ist.

Im folgenden wird die Erfindung durch Beispiele illustriert, die jedoch nicht einschränkend verstanden werden sollen.

### Beispiel

Zehn Intensivstations-Patienten mit septischem Schock wurden jeweils zweimal mit dem erfindungsgemäßen Verfahren behandelt. Nach der Identifikation geeigneter Patienten wurde durch die Transfusionsmedizinische Abteilung der Klinik ein zum Patienten jeweils blutgruppen-gleicher Blutspender zur Spende eingeladen. Nach Vorstimulation des Spenders mit Methylprednisolon wurde am darauffolgenden Tag die Entnahme der Spenderzellen durchgeführt (Dichtegradienten-Zentrifugation mit Hydroxyaethylstärke (HAES) /Zitrat nach Standard-Protokoll der Transfusionsmedizin zur Granulozytenspende). Die derart gewonnenen zwanzig Zellspenden, die zwischen 1,4 und 3,4 x 10¹⁰ Granulozyten pro Liter, zwischen 1,5 und 4,5 x 10¹² Erythrozyten pro Liter und zwischen 1,3 und 7, 1 x 10¹¹ Thrombozyten pro Liter enthielten, d.h. das Bioäquivalent, wurden bestrahlt, und dann wurde unmittelbar die Behandlung für sechs Stunden am Patientenbett durchgeführt.

Dazu wurden die Zellen in ein Bioreaktorsystem eingefüllt, in dem die Zellen durch ein Schlauch- und Gefäßsystem kreisen konnten (angetrieben durch eine Schlauch-Rollenpumpe) (Fig.1). In einem zweiten Teil der Apparatur wurde Blut des Sepsis-Patienten durch einen Plasmafilter geleitet, wodurch ein Teil des Blutplasmas aus dem Blutfluss abgetrennt wurde (Plasmaseparation). Dieses abgetrennte Plasma wurde on-line dem kreisenden Bioäquivalent zugefügt. Im gleichen Volumen-je Zeit-Verhältnis wird aus dem Bioäquivalent Plasma durch Plasmaseparation (mittels eines zweiten Plasmafilters) abgetrennt und dem Patientenblut wieder zugeführt. Zur Vermeidung der Blutgerinnung im extrakorporalen System wurde eine Antikoagulation mit Heparin durchgeführt. Das im Bioreaktorsystem befindliche Blut/Plasma/Bioäquivalent wurde durch Geräte-Heizung vor dem übermäßigen Auskühlen bewahrt (d.h. auf eine Temperatur von ca. 34-37°C gehalten). Das Patientenblut floß mit 150-200 ml/min, der Plasmafluß betrug ca. 20-35 ml/min, das Bioäquivalent kreiste mit 200 ml/min. Nach sechs Stunden wurde die Behandlung beendet. Das Bioreaktorsystem wurde verworfen, das Bioäquivalent wurde ebenfalls verworfen oder zur Labor-Analyse verwendet. Die zweite Behandlung fand nach einem Tag Pause statt (d.h. zwei Behandlungen innerhalb von 72 Stunden) und wurde analog der ersten durchgeführt.

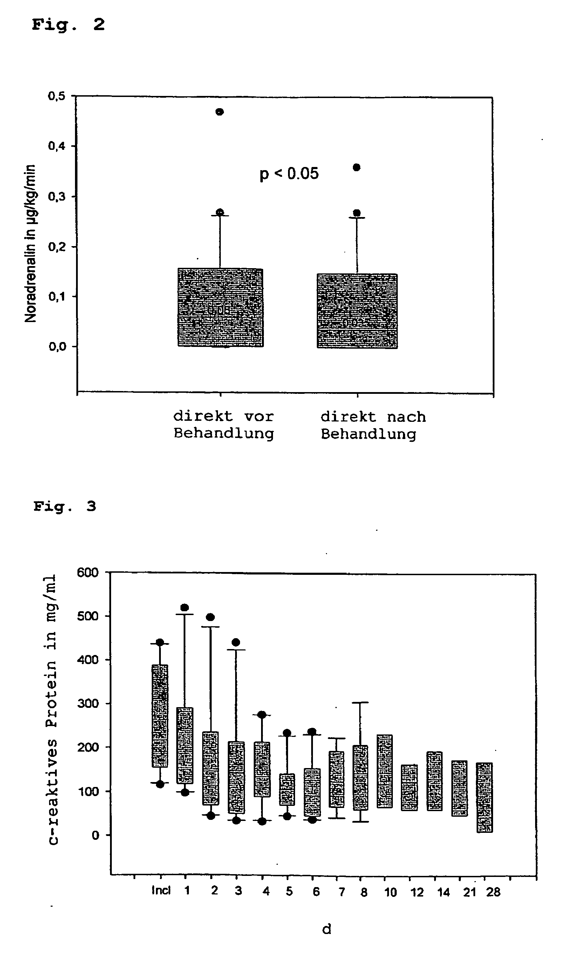

Alle Patienten haben die Behandlungen gut vertragen. Überraschenderweise besserte sich noch während der Behandlung die Kreislaufschock-Situation der Patienten insofern, als daß die medikamentöse Kreislaufunterstützung mit Katecholaminen signifikant reduziert werden konnte (Fig.2).

Im Verlauf der folgenden 28 Tage besserte sich die septische Schock und Multiorganversagens-Situation bei den meisten Patienten (fallende Entzündungsmarker, z.B. für C-Reaktives Protein, Procalcitonin, s. Fig. 3, 4). Die zelluläre Immunkompetenz der Patienten verbesserte sich signifikant (Anstieg der HLA-DR-Werte je Monozyt, Fig.5). Schließlich nahm die Anzahl und Schwere der assoziierten Organversagen ab (fallender Organversagens-Score SOFA, Fig. 6).

Die Überlebensraten waren - verglichen mit den überlebensprädiktiven Scores überraschend hoch: sieben Patienten überlebten für mind. 28 Tage und sechs konnten aus dem Krankenhaus entlassen werden. Durch den Acute Physiology and Chronic Health Evaluation (APACHE) II-Score vorhergesagt waren nur 2-3 Krankenhaus-Überlebende.

### Legende der Abbildungen

**Fig. 1****:** Schematische Darstellung einer bevorzugten erfindungsgemäßen Behandlungsapparatur (dargestellt beim Befüllen der Apparatur). Dunkle Linie: Extrakorporaler Blutkreislauf des Patienten/Blutschlauch **(1)**. Helle Linie: Plasmafluß nach Plasmaseparation aus dem Patientenblut bzw. aus dem Bioäquivalent/Plasmaschlauch **(2)**. Gestrichelte Linie: Bioäquivalenzraum/Zellkreislaufschlauch **(3)**.

Cellbag **(4)** stellt die Quelle der Zellen des Bioäquivalents dar. Diese Verbindung wird während der Behandlung getrennt. BM11 **(5)** und BM14 **(6)** sind Geräte, die von der Firma Baxter/Edwards hergestellt werden (s.o.), und diese Geräte werden auch gemeinsam vertrieben und als BM25 bezeichnet, der MARS-Monitor **(7)** wird von Gambro, Rostock hergestellt. Es können jeweils auch andere Geräte verwendet werden, auch z.B. Zellzentrifugen, die dazu geeignet sind, Plasma von Zellen zu separieren. Die Geräte übernehmen neben der Filtration auch die Aufgabe, gegebenenfalls den Druck und/oder Temperatur zu überwachen und den Kreislauf anzutreiben (z.B. über Rollerpumpen). Die Temperierung kann z.B. über die Heizeinheit des MARS-Monitors realisiert werden (Heizbeutel **(8)**). Cellmodule **(9)** bezeichnet einen Expansions- und Sedimentationsraum für die Zellen. CellFilter I **(10)** und CellFilter II **(11)** sind bevorzugt Hohlfasermembran-Plasmafilter, die die Zellen des Bioäquivalents zurückhalten. Es kann z.B. PF1000N (Gambro) verwendet werden. CellFilter II ist nicht notwendig, sondern ist eine im Prinzip redundante Filtereinheit gleichen Aufbaus wie CellFilter I, die aus Sicherheitsgründen verwendet werden kann. Im Wastebag **(12)** wird beim Befüllen des Systems die in der Apparatur vorhandene und dann durch das Bioäquivalent ersetzte Flüssigkeit gesammelt. Diese Verbindung wird während der Behandlung getrennt.
**(13)** Zellinfusionsschlauch; **(14)** PF1000N; **(15)** Heparin; Priming-Lösung: heparinisierte HF-Lösung

**Fig. 2****:** Katecholamin-Bedarf bei Sepsis-Patienten vor und nach der erfindungsgemäßen Behandlung: Signifikanter Abfall des Noradrenalin-Bedarfes bei stabilem Blutdruck.

**Fig. 3****:** 28-Tage-Verlauf des C-reaktiven Protein (CRP)-Wertes bei zehn Patienten mit septischem Schock (ab dem Tag der Erstbehandlung mit dem erfindungsgemäßen Verfahren. d = Studientage). Signifikante Senkung von hochpathologischen Werten in Richtung Normalwerte über den abgebildeten Zeitraum.

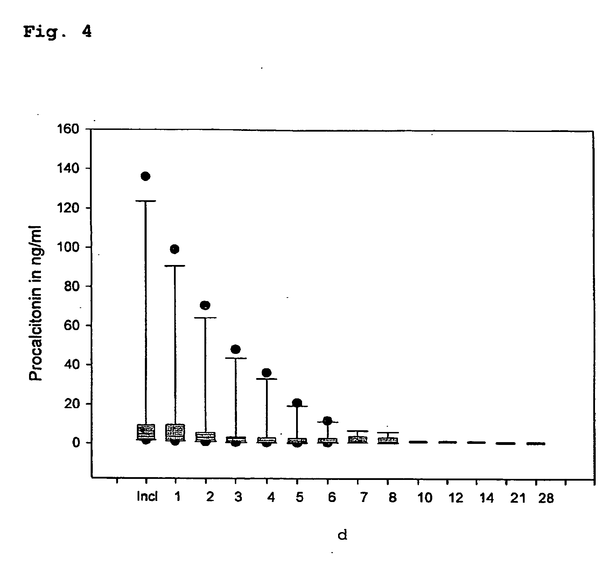

**Fig. 4****:** 28-Tage-Verlauf des Procalcitonin (PCT)-Wertes bei zehn Patienten mit septischem Schock (ab dem Tag der Erstbehandlung mit dem erfindungsgemäßen Verfahren). Signifikante Senkung von pathologischen Werten in den Normalwertbereich über den abgebildeten Zeitraum.

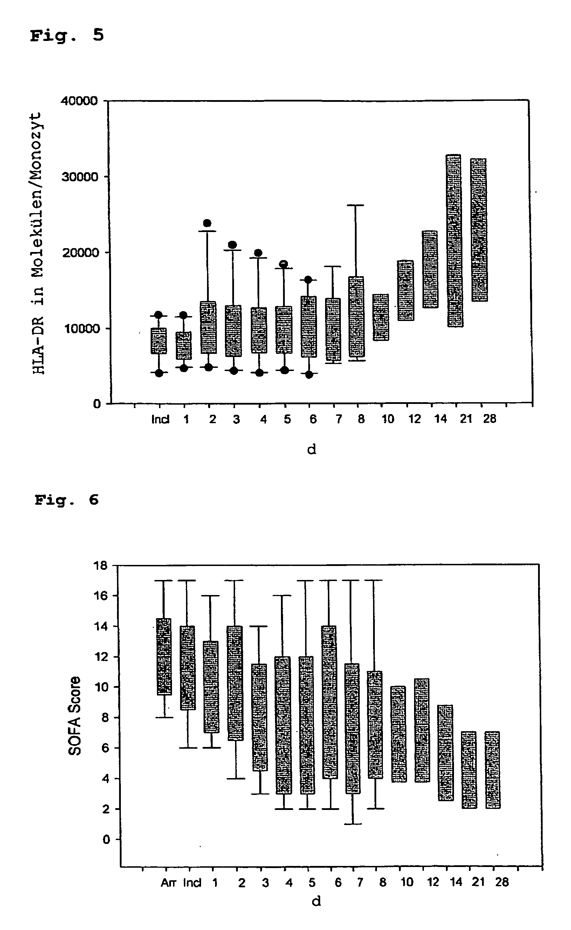

**Fig. 5****:** 28-Tage-Verlauf des Humanen Leukozyten-Antigens (HLA)-DR-Wertes (HLA-DR-Moleküle je Monozyt) bei zehn Patienten mit septischem Schock (ab dem Tag der Erstbehandlung mit dem erfindungsgemäßen Verfahren). Signifikanter Anstieg von pathologischen Werten in den Normalwertbereich über den abgebildeten Zeitraum.

**Fig. 6****:** 28-Tage-Verlauf des Sequential Organ Failure Assessment (SOFA)-Score-Wertes bei zehn Patienten mit septischem Schock (ab dem Tag der Erstbehandlung mit dem erfindungsgemäßen Verfahren). Signifikante Senkung von pathologischen Werten in Richtung Normalwertbereich über den abgebildeten Zeitraum.

### Referenzen

1. Thomas L (Hrsg) Labor und Diagnose. TH-Books Verlagsgesellsch.mbH, Frankfurt/Main, 5.Aufl. 2000 (ISBN 3-9805215-3-2)
2. Ronco C, Bellomo R (Hrsg.): Critical Care Nephrology. Kluwer Academic Publishers Dordrecht, Boston, London 1998 (ISBN 0-7923-4610-6)
3. van de Kerkhove MP et al. Clinical application of bioartificial liver support systems. Ann Surg. 2004;240:216-30
4. Mitzner SR. Albumin dialysis: an update. Curr Opin Nephrol Hypertens. 2007;16:589-95
5. Lehmann HC et al. Plasma exchange in neuroimmunological disorders: part 2. Treatment of neuromuscular disorders. Arch Neurol. 2006;63:1066-71
6. Blessing F et al. Heparin-mediated extracorporeal lowdensity lipoprotein precipitation: rationale for a specific adjuvant therapy in cardiovascular disease. Transfus Apher Sci. 2004;30:255-66
7. Bellomo R et al: Extracorporeal blood treatment (EBT) methods in SIRS/Sepsis. Int J Artif Organs 2005; 28:450-8
8. Ronco C, Inguaggiato P, D'Intini V, Cole L, Bellomo R, Poulin S, Bordoni V, Crepaldi C, Gastaldon F, Brendolan A, Trairak P, Khajohn T: The role of extracorporeal therapies in sepsis. J Nephrol. 16 Suppl 7:S34-S41, 2003.
9. Cole L, Bellomo R, Hart G, Journois D, Davenport P, Tipping P, Ronco C: A phase II randomized, controlled trial of continuous hemofiltration in sepsis. Crit Care Med. 30:100-106, 2002.
10. Busund R, Koukline V, Utrobin U, Nedashkovsky E: Plasmapheresis in severe sepsis and septic shock: a prospective, randomised, controlled trial. Intensive Care Med. 28(10):1434-1439, 2002.
11. Stegmayr BG, Banga R, Berggren L, Norda R, Rydvall A, Vikerfors T: Plasma exchange as rescue therapy in multiple organ failure including acute renal failure. Crit Care Med 31(6):1730-1736, 2003.
12. Ronco C, Brendolan A, Lonnemann G, Bellomo R, Piccinni P, Digito A, Dan M, Irone M, La Greca G, Inguaggiato P, Maggiore U, De Nitti C, Wratten ML, Ricci Z, Tetta C: A pilot study of coupled plasma filtration with adsorption in septic shock. Crit Care Med. 30(6):1250-1255, 2002.
13. Tetta C, Bellomo R, Ronco C: Artificial organ treatment for multiple organ failure, acute renal failure, and sepsis: recent new trends. Artif Organs 27(3):202-213, 2003.
14. Vale JA, Rees AJ, Widdop B, Goulding R: Use of charcoal haemoperfusion in the management of severely poisoned patients. Br med J I (1975) 5-9
15. O'Grady JG, Gimson AES, O'Brien CJ, Pucknell A, Hughes RD, Williams R: Controlled trials of charcoal hemoperfusion and prognostic factors in fulminant hepatic failure. Gastroenterol 94 (1988) 1186-1192
16. Weston MJ, Gazzard BG, Buxton BH, Winch J, Flax H, Machado AL, Williams R: Effects of haemoperfusion through charcoal or XAD-2 resin on an animal model of fulminant liver failure. Gut 15 (1974) 482-486
17. Opolon P, Rapin JR, Huguet C, Granger A, Delorme ML, Boschat M, Sausse A: Hepatic failure coma (HFC) treated by polyacrylonitrile membrane (PAN) hemodialysis (HD). Trans Am Soc Artif Intern Organs 22 (1976) 701-708
18. Konstantin P, Chang J, Otto V, Brunner G: Artificial liver. Artif Organs 16 (1992) 235- 242
19. Ash SR: Hemodiabsorption in treatment of acute hepatic failure and chronic cirrhosis with ascites. Artif Organs 18 (1994) 355-362
20. Bambauer R: Therapeutischer Plasmaaustausch und verwandte Plasmaseparationsverfahren. Schattauer, Stuttgart, New York, 1988, 182-189
21. Stanworth SJ, Massey E, Hyde C, Brunskill S, Lucas G, Navarrete C, Marks DI: Granulocyte transfusions for treating infections in patients with neutropenia or neutrophil dysfunction. Cochrane Database Syst Rev 2005 (3):CD005339, 2005
22. Safdar A, Hanna HA, Boktour M, Kontoyiannis DP, Hachem R, Lichtiger B, Freireich EJ, Raad II: Impact of high-dose granulocyte transfusions in patients with cancer with candidemia: retrospective case-control analysis of 491 episodes of Candida species bloodstream infections. Cancer 101:2859-2865, 2004.
23. Oberholzer A et al. Sepsis syndromes: understanding the role of innate and acquired immunity. Shock. 2001;16:83-96
24. Docke WD, Randow F, Syrbe U, Krausch D, Asadullah K, Reinke P, Volk HD, Kox W: Monocyte deactivation in septic patients: restoration by IFN-gamma treatment. Nat Med. 3(6):678-681, 1997.
25. Caille V, Chiche JD, Nciri N, Berton C, Gibot S, Boval B, Payen D, Mira JP, Mebazaa A: Histocompatibility leukocyte antigen-D related expression is specifically altered and predicts mortality in septic shock but not in other causes of shock. Shock 22:521-526, 2004.
26. Ploder M, Pelinka L, Schmuckenschlager C, Wessner B, Ankersmit HJ, Fuerst W, Redl H, Roth E, Spittler A: Lipopolysaccharide-induced tumor necrosis factor alpha production and not monocyte human leukocyte antigen-DR expression is correlated with survival in septic trauma patients. Shock 25:129-134, 2006.
27. Kaufmann I, Hoelzl A, Schliephake F, Hummel T, Chouker A, Peter K, Thiel M: Polymorphonuclear leukocyte dysfunction syndrome in patients with increasing sepsis severity. Shock 26:254-261, 2006.

## Patentansprüche

1. Ex vivo Verfahren zur Behandlung von Blutkompositionsstörungen und Blutfunktionsstörungen, Schritte umfassend, bei denen man Blut oder Blutplasma eines Patienten extrakorporal mit einer zweiten Flüssigkeit (Bioäquivalent) in Kontakt bringt, **dadurch gekennzeichnet, dass** die zweite Flüssigkeit Blut oder modifiziertes Blut ist, welches Granulozyten, Thrombozyten und Erythrozyten umfasst.

2. Verfahren nach Anspruch 1, **dadurch gekennzeichnet, dass** das Bioäquivalent mindestens 0,2-100 x 10¹⁰/l Granulozyten, mindestens 1,0 x 10⁹/1 Thrombozyten und mindestens 1,0 x 10⁹/1 Erythrozyten umfasst.

3. Verfahren nach einem der vorhergehenden Ansprüche, **dadurch gekennzeichnet, dass** das Bioäquivalent ferner Lymphozyten, und/oder Monozyten umfasst.

4. Verfahren nach einem der vorhergehenden Ansprüche, **dadurch gekennzeichnet, dass** das Bioäquivalent ferner Blutplasma und/oder physiologische Kochsalzlösung oder eine geeignete Pufferlösung umfasst.

5. Verfahren nach einem der vorhergehenden Ansprüche, **dadurch gekennzeichnet, dass** das Bioäquivalent Blut umfasst, das durch Volumeneinengung, Leukozytenfraktionsanreicherung, Leukozytendepletion, Erythrozytendepletion, Depletion von Antikörpern, Bestrahlung, Gerinnungshemmung und/oder Stabilisierung modifiziert ist.

6. Verfahren nach einem der vorhergehenden Ansprüche, **dadurch gekennzeichnet, dass** das Bioäquivalent ein Granulozytenkonzentrat oder ein Buffy Coat ist.

7. Verfahren nach einem der vorhergehenden Ansprüche, Schritte umfassend, bei denen man
a) aus Patientenblut gewonnenes Blutplasma gleichzeitig zu dessen Gewinnung oder zeitverzögert dem Bioäquivalent zuleitet,
b) durch einen Filtrationsschritt das modifizierte Blutplasma von den Zellen des Bioäquivalents trennt, damit es dem Patientenblut wieder zugeführt werden kann.

8. Verfahren nach Anspruch 7, **dadurch gekennzeichnet, dass** das Blutplasma aus Patientenblut durch Anlegen eines Druckgradienten über eine semipermeable Membran gewonnen wurde.

9. Verfahren nach den Ansprüchen 1-6, **dadurch gekennzeichnet, dass** Patientenblut oder Patientenblutplasma und das Bioäquivalent durch eine semipermeable, plasmadurchlässige Membran voneinander getrennt sind.

10. Verfahren nach den Ansprüchen 8-9, **dadurch gekennzeichnet, dass** die Membran mindestens auf der Seite, die mit dem Bioäquivalent in Kontakt steht, biokompatibel ist.

11. Verfahren nach einem der vorhergehenden Ansprüche, **dadurch gekennzeichnet, dass** das Patientenblut oder Patientenblutplasma in einem extrakorporalen Kreislauf gerichtet bewegt wird und/oder das Bioäquivalent gerichtet bewegt wird.

12. Verfahren nach einem der vorhergehenden Ansprüche, **dadurch gekennzeichnet, dass** das Patientenblut oder Patientenblutplasma in einem extrakorporalen Kreislauf von dem Kreislauf des Patienten getrennt ist.

13. Verfahren nach einem der vorhergehenden Ansprüche, **dadurch gekennzeichnet, dass** die Blutkompositionsstörung oder Blutfunktionsstörung Sepsis oder septischer Schock, insbesondere in der Phase der Immunparalyse, Niereninsuffizienz, Leberinsuffizienz, Immundefizienz oder eine Infektionskrankheit ist oder damit in Verbindung steht.

14. Vorrichtung, geeignet zur Durchführung eines Verfahren zur Behandlung von Blutkompositionsstörungen und Blutfunktionsstörungen, welches Schritte umfasst, bei denen man Blut oder Blutplasma eines Patienten extrakorporal mit einer zweiten Flüssigkeit (Bioäquivalent) in Kontakt bringt, wobei die zweite Flüssigkeit Blut oder modifiziertes Blut ist, welches Granulozyten, Thrombozyten und Erythrozyten umfasst, wobei die Vorrichtung zwei durch eine oder mehrere semipermeable Membranen getrennte Räume umfasst, von denen einer zur Aufnahme von Patientenblut oder Blutplasma geeignet ist und der zweite zur Aufnahme des Bioäquivalents geeignet ist.

15. Vorrichtung nach Anspruch 14, **dadurch gekennzeichnet, dass** das Volumen des zweiten Raums variabel ist und bevorzugt zwischen 100 mL und 20 L liegt.

16. Vorrichtung nach Anspruch 14 oder 15, **dadurch gekennzeichnet, dass** die Membran mindestens auf der Seite, die mit dem Bioäquivalent in Kontakt steht, biokompatibel ist.

17. Vorrichtung nach den Ansprüchen 14-16, **dadurch gekennzeichnet, dass** der zweite Raum Bioäquivalent umfasst.

18. Vorrichtung nach den Ansprüchen 14-17 zur Anwendung bei Behandlung einer Blutkompositionsstörung oder Blutfunktionsstörung.

19. Blut oder eine daraus gewonnenen Flüssigkeit, welche Granulozyten, Thrombozyten und Erythrozyten umfasst (Bioäquivalent), zur Anwendung bei Behandlung einer Blutkompositionsstörung oder Blutfunktionsstörung, **dadurch gekennzeichnet, dass** man Blut oder Blutplasma eines Patienten extrakorporal mit dem Bioäquivalent in Kontakt bringt.

## Claims

1. A method for the treatment of blood composition disorders and blood function disorders, comprising steps wherein blood or blood plasma of a patient is extracorporeally contacted with a second liquid (bioequivalent), **characterized in that** the second liquid is blood or modified blood comprising granulocytes, thrombocytes, and erythrocytes.

2. The method of claim 1, **characterized in that** the bioequivalent comprises at least 0.2-100 x 10¹⁰ granulocytes per L, at least 1.0 x 10⁹ thrombocytes per L, and at least 1.0 x 10⁹ erythrocytes per L.

3. The method of any of the preceding claims, **characterized in that** the bioequivalent further comprises lymphocytes and/or monocytes.

4. The method of any of the preceding claims, **characterized in that** the bioequivalent further comprises blood plasma and/or physiological saline solution or a suitable buffer solution.

5. The method of any of the preceding claims, **characterized in that** the bioequivalent comprises blood modified by volume restriction, leukocyte fraction enrichment, leukocyte depletion, erythrocyte depletion, depletion of antibodies, irradiation, coagulation inhibition, and/or stabilization.

6. The method of any of the preceding claims, **characterized in that** the bioequivalent is a granulocyte concentrate or a buffy coat.

7. The method of any of the preceding claims, comprising steps wherein
a) blood plasma obtained from patient's blood is fed to the bioequivalent either simultaneous to its acquisition or at a later time point,
b) the modified blood plasma is separated from the cells of the bioequivalent by means of a filtration step, so that it can be re-administered to the patient's blood.

8. The method of claim 7, **characterized in that** the blood plasma from patient's blood was obtained by setting up a pressure gradient over a semipermeable membrane.

9. The method of claims 1-6, **characterized in that** patient's blood or patient's blood plasma and the bioequivalent are separated from one another by a semipermeable, plasma-permeable membrane.

10. The method of claims 8-9, **characterized in that** the membrane is biocompatible at least on the side which is in contact with the bioequivalent.

11. The method of any of the preceding claims, **characterized in that** the patient's blood or patient's blood plasma is moved directionally in an extracorporeal circulation and/or the bioequivalent is moved directionally.

12. The method of any of the preceding claims, **characterized in that** the patient's blood or patient's blood plasma in an extracorporeal circulation is separated from the circulation of the patient.

13. The method of any one of the preceding claims 1, wherein the blood composition disorder or blood function disorder is sepsis or septic shock, in particular in the phase of immunoparalysis, renal insufficiency, liver insufficiency, immunodeficiency, or an infectious disease or is associated with it.

14. An apparatus suitable for carrying out a method for the treatment of blood composition disorders and blood function disorders, comprising steps wherein blood or blood plasma of a patient is extracorporeally contacted with a second liquid (bioequivalent), wherein the second liquid is blood or modified blood comprising granulocytes, thrombocytes, and erythrocytes, wherein the apparatus comprises two spaces separated by one or more semipermeable membrane(s), wherein one space is suitable for accepting patient's blood or blood plasma and the second space is suitable for accepting the bioequivalent.

15. The apparatus of claim 14, **characterized in that** the volume of the second space is variable and is preferably in the range from 100 mL to 20 L.

16. The apparatus of claim 14 or 15, **characterized in that** the membrane is biocompatible at least on the side which is in contact with the bioequivalent.

17. The apparatus of claims 14-16, **characterized in that** the second space comprises bioequivalent.

18. The apparatus of claims 14-17 for use in the treatment of a blood composition disorder or blood function disorder.

19. Blood or a liquid obtained therefrom, which comprises granulocytes, thrombocytes, and erythrocytes (bioequivalent), for use in the treatment of a blood composition disorder or blood function disorder, **characterized in that** blood or blood plasma of a patient is extracorporeally contacted with the bioequivalent.

## Revendications

1. Procédé *ex vivo* de traitement de troubles de composition du sang ou de troubles de fonction du sang, comportant des étapes dans lesquelles on met du sang ou du plasma sanguin d'un patient en contact extracorporel avec un deuxième liquide appelé "bioéquivalent", **caractérisé en ce que** ce deuxième liquide est du sang ou du sang modifié qui comprend des granulocytes, des thrombocytes et des érythrocytes.

2. Procédé conforme à la revendication 1, **caractérisé en ce que** le bioéquivalent comprend au moins de 0,2.10¹⁰ à 100.10¹⁰ granulocytes par litre, au moins 1,0.10⁹ thrombocytes par litre, et au moins 1,0.10⁹ érythrocytes par litre.

3. Procédé conforme à l'une des revendications précédentes, **caractérisé en ce que** le bioéquivalent comprend en outre des lymphocytes et/ou des monocytes.

4. Procédé conforme à l'une des revendications précédentes, **caractérisé en ce que** le bioéquivalent comprend en outre du plasma sanguin et/ou de la solution salée physiologique ou une solution tampon appropriée.

5. Procédé conforme à l'une des revendications précédentes, **caractérisé en ce que** le bioéquivalent comprend du sang qui a été modifié par concentration en volume, enrichissement en leucocytes, appauvrissement en leucocytes, appauvrissement en érythrocytes, appauvrissement en anticorps, irradiation, inhibition de la coagulation et/ou stabilisation.

6. Procédé conforme à l'une des revendications précédentes, **caractérisé en ce que** le bioéquivalent est un concentré de granulocytes ou une couche leucocytaire.

7. Procédé conforme à l'une des revendications précédentes, comprenant des étapes dans lesquelles
a) on ajoute au bioéquivalent du plasma sanguin obtenu à partir du sang du patient, simultanément à son obtention ou avec retard ;
b) et l'on sépare par filtration le plasma sanguin modifié d'avec les cellules du bioéquivalent, afin de pouvoir le renvoyer dans le sang du patient.

8. Procédé conforme à la revendication 7, **caractérisé en ce que** l'on obtient le plasma sanguin à partir du sang du patient en établissant un gradient de pression de part et d'autre d'une membrane semi-perméable.

9. Procédé conforme aux revendications 1 à 6, **caractérisé en ce que** le sang du patient ou le plasma sanguin du patient et le bioéquivalent sont séparés l'un de l'autre par une membrane semi-perméable qui laisse passer le plasma.

10. Procédé conforme aux revendications 8 à 9, **caractérisé en ce que** la membrane est biocompatible, au moins du côté qui est en contact avec le bioéquivalent.

11. Procédé conforme à l'une des revendications précédentes, **caractérisé en ce qu'**on fait circuler le sang du patient ou le plasma sanguin du patient dans un circuit extracorporel et/ou l'on fait circuler le bioéquivalent.

12. Procédé conforme à l'une des revendications précédentes, **caractérisé en ce qu'**il y a séparation entre le sang du patient ou le plasma sanguin du patient, qui circule dans un circuit extracorporel, et la circulation du patient.

13. Procédé conforme à l'une des revendications précédentes, **caractérisé en ce que** le trouble de composition du sang ou le trouble de fonction du sang est, ou est en relation avec, une septicémie ou un choc septique, en particulier dans la phase de paralysie immunitaire, une insuffisance rénale, une insuffisance hépatique, un déficit immunitaire ou une maladie infectieuse.

14. Dispositif, approprié pour la mise en oeuvre d'un procédé de traitement de troubles de composition du sang ou de troubles de fonction du sang, comportant des étapes dans lesquelles on met du sang ou du plasma sanguin d'un patient en contact extracorporel avec un deuxième liquide appelé "bioéquivalent", lequel deuxième liquide est du sang ou du sang modifié qui comprend des granulocytes, des thrombocytes et des érythrocytes, et lequel dispositif comporte deux zones séparées par une ou plusieurs membrane(s) semi-perméable(s), zones dont l'une est adaptée pour recevoir le sang du patient ou son plasma sanguin et l'autre est adaptée pour recevoir le bioéquivalent.

15. Dispositif conforme à la revendication 14, **caractérisé en ce que** le volume des deux zones est variable et se situe de préférence entre 100 mL et 20 L.

16. Dispositif conforme à la revendication 14 ou 15, **caractérisé en ce que** la membrane est biocompatible, au moins du côté qui est en contact avec le bioéquivalent.

17. Dispositif conforme aux revendications 14 à 16, **caractérisé en ce que** la deuxième zone comprend le bioéquivalent.

18. Dispositif conforme aux revendications 14 à 17, pour utilisation dans le traitement d'un trouble de composition du sang ou d'un trouble de fonction du sang.

19. Sang ou liquide obtenu à partir de sang et comprenant des granulocytes, des thrombocytes et des érythrocytes (appelé "bioéquivalent"), pour utilisation dans le traitement d'un trouble de composition du sang ou d'un trouble de fonction du sang, **caractérisé en ce que** du sang ou du plasma sanguin d'un patient est mis en contact extracorporel avec ce bioéquivalent.
